# EUROPEAN PATENT APPLICATION

(11) **EP 4 538 262 A1**
(43) Date of publication of application: **16.04.2025**
(21) Application number: 23203278.9
(22) Date of filing: 12.10.2023
(51) Int. Cl.: C07D 401/04, C07C 57/15, C07C 59/255, A61P 35/00, A61K 31/53

(54) **COCRYSTALS OF VORASIDENIB**

(71) Applicant: SANDOZ AG, 4051 Basel (CH)
(72) Inventor: The designation of the inventor has not yet been filed
(74) Representative: Stefánsson, Stefán Einar

(57) **Abstract**

The present invention relates to vorasidenib solid-state forms, to a pharmaceutical composition comprising them, and to processes for their preparation.

## Description

### FIELD OF THE INVENTION

In a first aspect thereof, the present invention relates to a co-crystal of vorasidenib and a dicarboxylic acid, in particular to a cocrystal of vorasidenib and a saturated dicarboxylic acid, preferably a saturated C2-C6 dicarboxylic acid, more preferably a saturated C2-C4 dicarboxylic acid, even more preferably tartaric acid (e.g., D-tartaric acid and/or L-tartaric acid), glutaric acid and malonic acid, and methods for its preparation. In another embodiment thereof, the first aspect of the invention relates to a cocrystal of vorasidenib and an unsaturated dicarboxylic acid having E-configuration at the double bond, preferably an (E)-unsaturated C2-C6 dicarboxylic acid, more preferably an (E)-unsaturated C2-C4 dicarboxylic acid, even more preferably fumaric acid, and methods for its preparation. In yet another aspect, the present invention relates to a crystalline hydrate form of vorasidenib comprising less than 2.8 moles of water per mole of vorasidenib, preferably comprising from 0.5 to 2.5 moles of water per mole of vorasidenib, more preferably comprising from 1.0 to 2.2 moles of water per mole of vorasidenib, even more preferably comprising from 1.5 to 2.1 moles of water per mole of vorasidenib, most preferably to a dihydrate form of vorasidenib, and methods for its preparation. Furthermore, the invention relates to a pharmaceutical composition comprising at least one of the cocrystals and/or solid-state forms of the invention, preferably in an effective and/or predetermined amount, and at least one pharmaceutically acceptable excipient. The co-crystal, solid-state form and pharmaceutical composition of the present invention can be used as a medicament, in particular for the treatment of cancer, preferably of malignant glioma and/or myoblastoma.

### BACKGROUND OF THE INVENTION

Isocitrate dehydrogenases (IDHs) catalyze the oxidative decarboxylation of isocitrate to 2-oxoglutarate (i.e., α-ketoglutarate). These enzymes belong to two distinct subclasses, one of which utilizes NAD(+) as the electron acceptor and the other NADP(+). Five isocitrate dehydrogenases have been reported: three NAD(+)-dependent isocitrate dehydrogenases, which localize to the mitochondrial matrix, and two NADP(+)-dependent isocitrate dehydrogenases, one of which is mitochondrial and the other predominantly cytosolic. Each NADP(+)-dependent isozyme is a homodimer.

IDH1 (isocitrate dehydrogenase 1 (NADP+), cytosolic) catalyzes the oxidative decarboxylation of isocitrate to α-ketoglutarate. It has been discovered that mutations of IDH1 present in certain cancer cells result in a new ability of the enzyme to catalyze the NADPH-dependent reduction of α-ketoglutarate to (*R*)-2-hydroxyglutarate (2HG). The production of 2HG is believed to contribute to the formation and progression of cancer (Dang, L et al., Nature 2009, 462:739-44).

IDH2 (isocitrate dehydrogenase 2 (NADP+), mitochondrial) catalyzes the oxidative decarboxylation of isocitrate to α-ketoglutarate (α-KG). It has been discovered that mutations of IDH2 present in certain cancer cells result in a new ability of the enzyme to catalyze the NADPH-dependent reduction of α-ketoglutarate to (*R*)-2-hydroxyglutarate (2HG). 2HG is not formed by wild-type IDH2. The production of 2HG is believed to contribute to the formation and progression of cancer (Dang, L et al, Nature 2009, 462:739-44).

U.S. Publication No. 2015/0018328 A1 discloses a compound described by the chemical name 6-(6-chloropyridin-2-yl)-*N*²,*N*⁴-bis((*R*)-1,1,1-trifluoropropan-2-yl)-1,3,5-triazine-2,4-diamine (INN: vorasidenib),and its chemical formula (I):

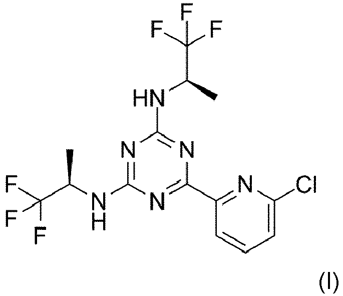

Vorasidenib (I) is described as a dual inhibitor of mutant IDH1 and IDH2 proteins in biochemical and cellular assays, meaning that it prevents the formation and accumulation of the oncometabolite 2HG, that occurs when genetically altered versions of two enzymes, IDH1 and IDH2, are present in a tumor. 2HG is thought to be responsible for the formation and maintenance of IDH-mutant gliomas.

Different solid-state forms of an active pharmaceutical ingredient (API) often possess different physical and chemical properties such as but not limited to dissolution rate, solubility, chemical stability, physical stability, hygroscopicity, melting point, morphology, flowability, bulk density and compressibility. Apart from solid-state forms of an API (such as polymorphs, pseudopolymorphs (hydrates and solvates) and salts), pharmaceutical cocrystals open up further opportunities for customizing the physicochemical properties of APIs with a process or clinical need. For example, these physicochemical properties can be tailored to enhance drug product bioavailability, stability and/or processability of APIs during drug product manufacture.

Cocrystals are structurally readily distinguishable from salts because unlike salts, their components are in a neutral state and interact nonionically. Cocrystals are structurally more similar to solvates and hydrates, in that both contain more than one component in the crystal lattice and the interaction between these components is nonionic. From a physico-chemical perspective, cocrystals can be viewed as a special case of solvates and hydrates, wherein the second component, the cocrystal former (coformer), is nonvolatile (see also "Regulatory Classification of Pharmaceutical Cocrystals", Guidance for Industry, FDA, Revision 1, August 2016).

WO2019090059A1 discloses (1) different solid forms of vorasidenib (Free Form Type A, Free Form Type B, Free Form Type C, Free Form Type D), (2) co-crystals with citric acid (Type A) and with maleic acid (Type A) and (3) methods for their preparation.

It is an objective of the present invention to provide an improved cocrystal or solid-state form of vorasidenib with improved dissolution, solubility, flowability, bulk density and/or compressibility properties and which can be anhydrous, non-hygroscopic, physically stable, chemically stable, and/or essentially free from organic solvents.

### SUMMARY OF THE INVENTION

The invention solves one or more of the above defined objectives by providing (1) a cocrystal of vorasidenib and a dicarboxylic acid, and/or (2) a crystalline hydrate form of vorasidenib comprising less than 2.8 moles of water per mole of vorasidenib. The cocrystal and/or solid-state form of the present invention, is characterized by one or more advantageous physicochemical properties, e.g., improved dissolution rate, solubility, chemical stability, physical stability, stability under humidity stress, melting point, morphology, flowability, bulk density and/or compressibility.

In a first aspect thereof, the present invention relates to a cocrystal of vorasidenib and a dicarboxylic acid.

In a first embodiment thereof, the fist aspect of the invention relates to cocrystal of vorasidenib and a saturated dicarboxylic acid, preferably a saturated C2-C6 dicarboxylic acid, more preferably a saturated C2-C4 dicarboxylic acid, even more preferably tartaric acid (e.g., D-tartaric acid and/or L-tartaric acid), glutaric acid and malonic acid.

In a second embodiment thereof, the fist aspect of the invention relates to cocrystal of vorasidenib and an unsaturated dicarboxylic acid having E-configuration at the double bond, preferably an (E)-unsaturated C2-C6 dicarboxylic acid, more preferably an (E)-unsaturated C2-C4 dicarboxylic acid, even more preferably fumaric acid.

In a second aspect thereof, the present invention relates to a crystalline hydrate form of vorasidenib comprising less than 2.8 molecules of water per molecule of vorasidenib, preferably comprising from 0.5 to 2.5 moles of water per mole of vorasidenib, more preferably comprising from 1.0 to 2.2 moles of water per mole of vorasidenib, even more preferably comprising from 1.5 to 2.1 moles of water per mole of vorasidenib, most preferably to a dihydrate form of vorasidenib.

In third aspect thereof, the present invention relates to a method for preparing (1) said cocrystal of vorasidenib and a dicarboxylic acid, and/or (2) said crystalline hydrate of vorasidenib.

In further aspects thereof, the present invention relates to (a) a pharmaceutical composition comprising at least one of the cocrystals and/or solid-state forms of the invention, preferably in an effective and/or predetermined amount, and at least one pharmaceutically acceptable excipient, and (b) a method of treating cancer, comprising administering said cocrystal, solid-state form and/or pharmaceutical composition of the invention, to a patient.

### Definitions

In the context of the present invention the following abbreviations have the indicated meaning, unless explicitly stated otherwise:
- API: active pharmaceutical ingredient
- DSC: differential scanning calorimetry
- DVS: dynamic vapor sorption
- H₂O: water
- m: mass
- PXRD: powder X-ray diffraction/diffractogram
- RH: relative humidity
- RT: room temperature
- SXRD: single crystal X-ray diffraction/diffractogram
- TGA: thermogravimetric analysis
- Vol/v: volume
- w: weight

The term "vorasidenib" as used herein refers to 6-(6-chloropyridin-2-yl)-*N*²,*N*⁴-bis((*R*)-1,1,1-trifluoropropan-2-yl)-1,3,5-triazine-2,4-diamine and characterized by formula (I).

The term "solid-state form" as used herein refers to any crystalline and/or amorphous phase of a compound. Crystalline phases include anhydrous, hydrate, solvate, or non-solvated forms of a given compound, as well as all the polymorphs of any one of them.

The term "cocrystal" as used herein refers to a crystalline material composed of two or more different molecules, typically active pharmaceutical ingredient (API) and at least one co-crystal former ("coformer") in the same crystal lattice wherein said molecules interact non-ionically, and wherein the at least one coformer is not a solvent and is typically non-volatile.

A cocrystal/solid-state form may be referred to herein as "substantially free" of any other solid-state form and/or co-crystal. As used herein, a cocrystal and/or a solid-state form of vorasidenib described herein as substantially free of any other cocrystal and/or solid-state form should be understood as containing at least about 80% (w/w), preferably at least about 90% (w/w), more preferably at least about 95% (w/w), even more preferably at least about 98% (w/w), most preferably at least about 99% (w/w), (e.g., at least about 99.5% (w/w), or about 99.9% (w/w)) of the cocrystal and/or solid-state form of vorasidenib, with respect to the total amount of the cocrystals and/or solid state forms present, as measured, for example, by PXRD. In some embodiments, the cocrystal and/or solid-state form of vorasidenib, may contain from about 1% to about 20% (w/w), preferably from about 2% to about 15% (w/w), more preferably from about 5% to about 10% (w/w) of other cocrystals and/or solid-state forms of vorasidenib, e.g., Free Form Type A, Free Form Type B, Free Form Type C, Free Form Type D or Citric Acid Cocrystal Type A.

A cocrystal, or a solid-state form, may be referred to herein as being characterized by graphical data "as depicted in" or "as substantially depicted in" a Figure, and should be understood to include any cocrystal and/or solid-state form of vorasidenib that is characterized by the depicted graphical data and, possibly, some experimental variations, as generally understood by a person skilled in the art, and deriving from, e.g., instrumentation used, humidity, season, pressure, temperature, degree of crystallinity, preferred orientation phenomena, and/or sample preparation.

As used herein, where a powder X-ray diffraction pattern (PXRD) refers to a group of specified peak positions, a margin of error ±0.2 degrees 2-theta (preferably ±0.1 degrees 2-theta, more preferably ±0.05 degrees 2-theta) shall be understood to apply to each peak position within the group.

The terms "vorasidenib cocrystal and a dicarboxylic acid" or "cocrystal of vorasidenib and a dicarboxylic acid" or "vorasidenib dicarboxylic acid cocrystal", as used interchangeably herein, refer to a crystalline compound comprising vorasidenib, as active pharmaceutical ingredient (API), and a dicarboxylic acid, as coformer, wherein the interaction between vorasidenib and the dicarboxylic acid is of nonionic and noncovalent nature.

As used herein, and unless stated otherwise, the term "non-solvated" or "anhydrous", as used herein, indicates that no organic solvent or water is cooperated in or accommodated by the crystal structure. Non-solvated or anhydrous forms may still contain residual organic solvents and/or water, which are not part of the crystal structure but may be adsorbed on the surface or absorbed in disordered regions of the crystal. Typically, a non-solvated or anhydrous form does not contain more than 1.0% (w/w), preferably not more than 0.5 % (w/w) of organic solvents or water, based on the weight of the cocrystal and/or solid-state form of vorasidenib. The organic solvent content can be determined by thermogravimetric analysis (TGA), e.g., by determining the mass loss in the range of from 25 to 280°C at a heating rate of 10 K/min or by 1H-NMR. The water content can be determined by Karl-Fischer Coulometry and/or by thermogravimetric analysis (TGA), e.g., by determining the mass loss in the range of from 25 to 280 °C at a heating rate of 10 K/min.

The term "solvated" as used herein indicates that organic solvent is cooperated in or accommodated by the crystal structure. In the specific case of water as the solvent, the form is usually referred to as a "hydrate".

The term "non-hygroscopic", as used herein, refers to a compound showing a mass change of less than 2% (based on the weight of the cocrystal and/or solid-state form of vorasidenib) when subjected to a relative humidity (RH) from 0 to 95% at 25.0 (± 0.1) °C.

The term "reflection" with regard to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to common general knowledge, long-range order normally extends over approximately 100 to 1000 atoms, whereas short-range order is over a few atoms only.

The term "essentially the same" with reference to powder X-ray diffraction means that variabilities in reflection positions and relative intensities of the reflections are to be taken into account. A typical precision of the 2-theta (2Θ) values is in the range of ± 0.2° 2Θ, preferably in the range of ± 0.1° 2Θ, more preferably in the range of ± 0.05° 2Θ. Thus, a reflection that appears at 15.3° 2Θ can appear from 15.1° to 15.5° 2Θ, preferably from 15.2° to 15.4° 2Θ, more preferably from 15.25° to 15.35° 2Θ, on most X-ray diffractometers under standard conditions. Furthermore, one skilled in the art will appreciate that relative reflection intensities will show inter-apparatus variability as well as variability due to degree of crystallinity, preferred orientation phenomena, sample preparation and other factors known to those skilled in the art.

As used herein, unless stated otherwise, the PXRD measurements are taken using Cu-Kalpha_{1,2} radiation (wavelength 1.5419 Å) at ambient temperature.

As used herein, unless stated otherwise, the SXRD measurements are taken using Mo radiation (wavelength 0.71073 Å) at -80 °C.

As used herein, unless stated otherwise, DSC analysis is carried out at a heating rate of 10 °C/min, preferably with a nitrogen flow of 20 mL/minute (sample purge).

As used herein, unless stated otherwise, TGA analysis is carried out at a heating rate of 10 °C/min, preferably with a nitrogen flow of 40 mL/minute (balance purge).

The amount of solvent employed in a chemical process, e.g., a reaction or crystallization, may be referred to herein as "volumes" or "vol" or "v." For example, a material may be referred to as being suspended in 10 volumes (or 10 vol or 10 v) of a solvent. In this context, this expression would be understood to mean milliliters (mL) of the solvent per gram (g) of the material being suspended, such that suspending 5 grams of a material in 10 volumes of a solvent means that the solvent is used in an amount of 10 milliliters per gram of the material that is being suspended or, in this example, 50 mL of the solvent. In another context, the term "v/v" may be used to indicate the number of volumes of a solvent with respect to the volume of a liquid mixture. For example, adding solvent X (1.5 v/v) to a 100 mL reaction mixture would indicate that 150 mL of solvent X was added.

A process, or a step, may be referred to herein as being carried out "overnight". This refers to a time interval that spans the time during the night, when that process, or step, may not be actively observed. As used herein, "overnight" normally refers to a time interval from about 8 to about 20 hours, or from about 10 to about 18 hours, in some cases from about 12 to about 16 hours.

As used herein, the term "reduced pressure" refers to a pressure that is less than the atmospheric pressure (about 1000 mbar). For example, reduced pressure is from about 10 mbar to about 50 mbar.

As used herein the term "room temperature" indicates that the applied temperature is not critical and that the exact value may not have been actively monitored. Usually, "room temperature" is understood to mean a temperature from about 15 to about 35 °C.

The cocrystal and/or solid-state form of vorasidenib of the present invention may be characterized by analytical methods well known in the field of the pharmaceutical industry for characterizing crystalline solids. Such methods comprise, but are not limited to, powder and single X-ray diffraction (PXRD), TGA and/or DSC. In particular, the cocrystal and solid-state forms of the present invention may be characterized by any one of the following embodiments or a combination thereof.

As used herein, the term "unit cell" refers to the smallest group of particles (e.g., molecules) in a crystalline solid that makes up the repeating pattern of the crystalline solid. In a cocrystal, the term "unit cell" refers to the smallest group of the two or more neutral chemical species that makes up the repeating pattern of the cocrystal.

### BRIEF DESCRIPTION OF THE FIGURES

**Figure 1****:** illustrates a representative PXRD of vorasidenib L-tartaric acid cocrystal Form 1. The x-axis shows the scattering angle in °2-Theta, the y-axis shows the intensity of the scattered X-ray beam in counts of detected photons.
**Figure 2****:** illustrates a representative PXRD of vorasidenib L-tartaric acid cocrystal Form 2. The x-axis shows the scattering angle in °2-Theta, the y-axis shows the intensity of the scattered X-ray beam in counts of detected photons.
**Figure 3****:** illustrates a representative PXRD of vorasidenib L-tartaric acid cocrystal Form 3. The x-axis shows the scattering angle in °2-Theta, the y-axis shows the intensity of the scattered X-ray beam in counts of detected photons.
**Figure 4****:** illustrates a representative DSC curve of vorasidenib L-tartaric acid cocrystal Form 3. The x-axis shows the temperature in degree Celsius (°C), the y-axis shows the heat flow rate in Watt per gram (W/g) with endothermic peaks going up.
**Figure 5****:** illustrates a representative TGA curve of vorasidenib L-tartaric acid cocrystal Form 3. The x-axis shows the temperature in degree Celsius (°C), the y-axis shows the mass (loss) of the sample in weight percent (weight%).

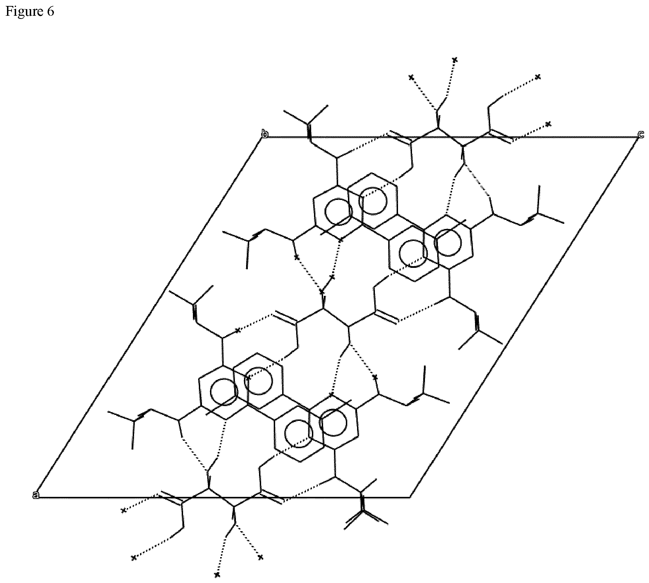

**Figure 6****:** illustrates a unit cell diagram of the single crystal of vorasidenib L-tartaric acid cocrystal Form 3, prepared as described in Example 5.
**Figure 7****:** illustrates the solubility curves of vorasidenib L-tartaric acid cocrystal Form 3, vorasidenib Free Form Type C (trihydrate), vorasidenib malonic acid cocrystal and vorasidenib citric acid cocrystal Type A.
**Figure 8****:** illustrates a representative PXRD of vorasidenib L-tartaric acid cocrystal Form 4. The x-axis shows the scattering angle in °2-Theta, the y-axis shows the intensity of the scattered X-ray beam in counts of detected photons.
**Figure 9****:** illustrates a representative PXRD of vorasidenib L-tartaric acid cocrystal Form 5. The x-axis shows the scattering angle in °2-Theta, the y-axis shows the intensity of the scattered X-ray beam in counts of detected photons.
**Figure 10****:** illustrates a representative PXRD of vorasidenib L-tartaric acid cocrystal Form 6. The x-axis shows the scattering angle in °2-Theta, the y-axis shows the intensity of the scattered X-ray beam in counts of detected photons.
**Figure 11****:** illustrates a representative PXRD of vorasidenib malonic acid cocrystal. The x-axis shows the scattering angle in °2-Theta, the y-axis shows the intensity of the scattered X-ray beam in counts of detected photons.
**Figure 12****:** illustrates a representative PXRD of vorasidenib glutaric acid cocrystal. The x-axis shows the scattering angle in °2-Theta, the y-axis shows the intensity of the scattered X-ray beam in counts of detected photons.
**Figure 13****:** illustrates a representative PXRD of vorasidenib fumaric acid cocrystal. The x-axis shows the scattering angle in °2-Theta, the y-axis shows the intensity of the scattered X-ray beam in counts of detected photons.
**Figure 14****:** illustrates a representative DSC curve of vorasidenib fumaric acid cocrystal. The x-axis shows the temperature in degree Celsius (°C), the y-axis shows the heat flow rate in Watt per gram (W/g) with endothermic peaks going up.
**Figure 15****:** illustrates a representative TGA curve of vorasidenib fumaric acid cocrystal. The x-axis shows the temperature in degree Celsius (°C), the y-axis shows the mass (loss) of the sample in weight percent (weight%).
**Figure 16****:** illustrates a representative DVS curve of vorasidenib fumaric acid cocrystal. The x-axis shows the relative humidity in percent (%), the y-axis shows the mass (water uptake) of the sample in weight percent (weight%).
**Figure 17****:** illustrates a representative PXRD of vorasidenib hydrate. The x-axis shows the scattering angle in °2-Theta, the y-axis shows the intensity of the scattered X-ray beam in counts of detected photons.
**Figure 18****:** illustrates a representative PXRD of vorasidenib D/L-tartaric acid cocrystal. The x-axis shows the scattering angle in °2-Theta, the y-axis shows the intensity of the scattered X-ray beam in counts of detected photons.

### DETAILED DESCRIPTION OF THE INVENTION

The present disclosure relates to (1) a cocrystal of vorasidenib and a dicarboxylic acid, (2) a crystalline hydrate form of vorasidenib, (3) drug substances and pharmaceutical compositions comprising (1) and/or (2), (4) methods for preparing (1) and (2) and/or (3), and (5) use of (1), (2) and/or (3) in therapy.

In a first aspect thereof, the present invention provides a cocrystal comprising vorasidenib, as the active pharmaceutical ingredient, and a dicarboxylic acid, preferably a C2-C6 dicarboxylic acid, more preferably a C2-C4 dicarboxylic acid, as the co-former.

In a first embodiment thereof, the first aspect of the invention provides a cocrystal comprising vorasidenib, as the active pharmaceutical ingredient, and a saturated dicarboxylic acid, preferably a saturated C2-C6 dicarboxylic acid, more preferably a saturated C2-C4 dicarboxylic acid, even more preferably tartaric acid (e.g., D-tartaric acid and/or L-tartaric acid), glutaric acid or malonic acid, as the co-former.

In a preferred embodiment thereof, the first aspect of the invention relates to a cocrystal of vorasidenib and L-tartaric acid of formula (II). 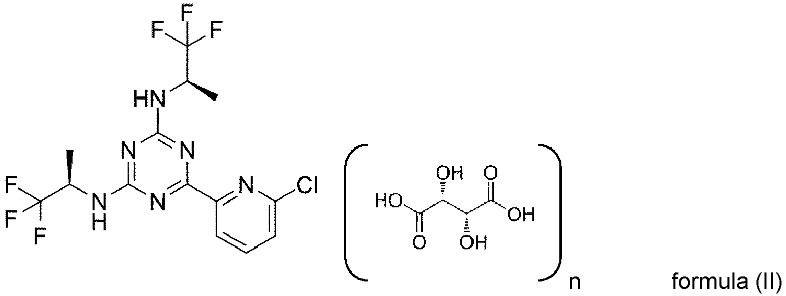 wherein n is from 0.2 to 0.8, preferably from 0.3 to 0.7, more preferably from 0.4 to 0.6, most preferably 0.5.

Preferably the cocrystal of vorasidenib with L-tartaric acid, hereinafter Form 3, is characterized by a PXRD comprising any one of the following list of reflections:
❖ (5.8 + 0.2) °, (10.2 + 0.2)° and (13.7 + 0.2)°; or
❖ (5.8 + 0.2) °, (10.2 + 0.2)°, (13.7 + 0.2)° and (19.1+ 0.2)°; or
❖ (5.8 + 0.2) °, (8.6 + 0.2)°, (10.2 + 0.2)°, (13.7 + 0.2)°, and (19.1 + 0.2)°; or
❖ (5.8 + 0.2) °, (8.6 + 0.2)°, (10.2 + 0.2)°, (13.7 + 0.2)°, (15.8 + 0.2)°, and (19.1 + 0.2)°; or
❖ (5.8 + 0.2) °, (8.6 + 0.2)°, (10.2 + 0.2)°, (13.7 + 0.2)°, (15.8 + 0.2)°, (19.1 + 0.2)°, and (27.4 + 0.2)°; or
when measured at RT with Cu-Kalpha1 radiation having a wavelength of 0.15419 nm.

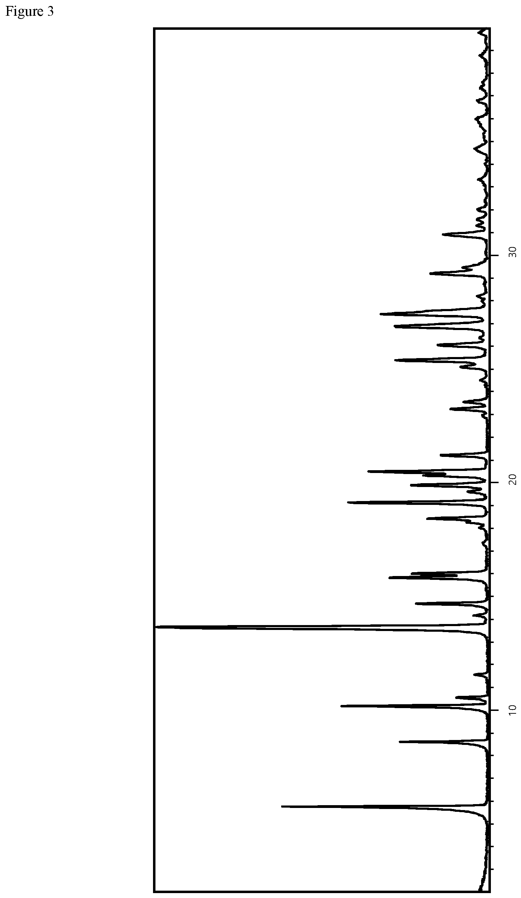

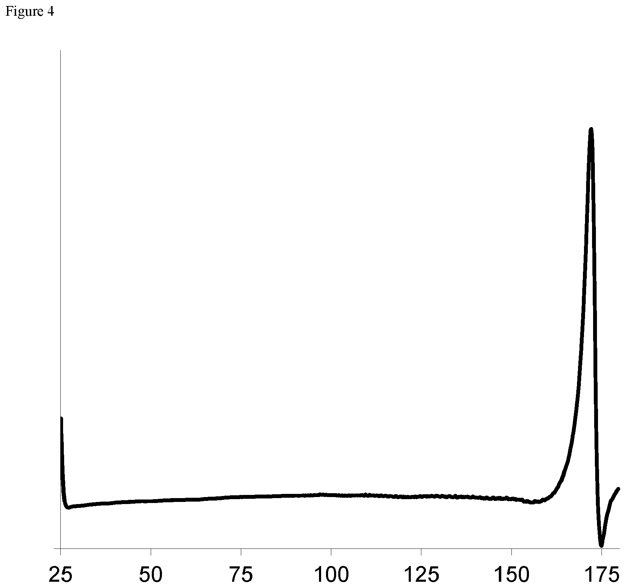

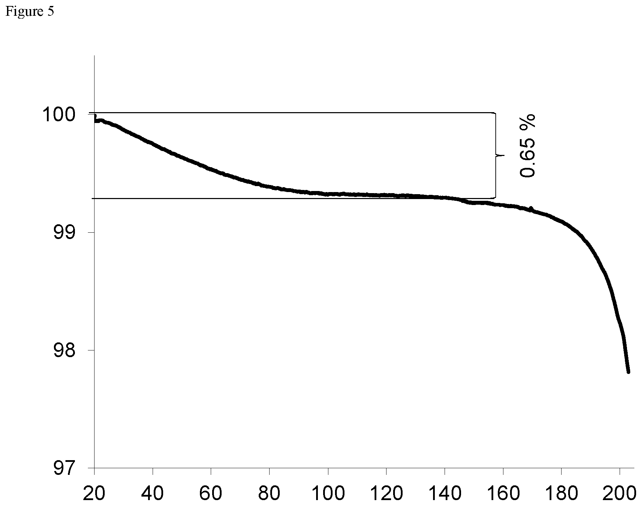

More preferably Form 3 is further characterized by at least one of the following features:
> a PXRD essentially as shown in figure 3, when measured at RT with Cu-Kalpha_{1,2} radiation having a wavelength of 0.15419 nm, and/or
> a monoclinic unit cells having space group C2 with the following parameters:
   a = 20.509(9)
   b = 6.9858(11)
   c = 18.099(8)
   alpha = 90°
   beta = 122.43(6)°
   gamma = 90°
when measured with single crystal X-ray diffraction at (173 ± 2) K with Cu-Kalpha1,2 radiation having a wavelength of 1.54184 Angstrom, and/or
> a DSC curve comprising an endothermic peak, preferably a single endothermic peak, having an onset temperature of (167 ± 5) °C, preferably of (167 ± 3) °C, more preferably of (167 ± 2) °C, even more preferably of (167 ± 1) °C, when measured at a heating rate of 10 K/min in a closed capsule, and/or
> a DSC curve comprising an endothermic peak, preferably a single endothermic peak, having a peak temperature of (170 ± 5) °C, preferably of (170 ± 3) °C, more preferably of (170 ± 2) °C, even more preferably of (170 ± 1) °C, when measured at a heating rate of 10 K/min in a closed capsule, and/or
> a DSC trace that, when measured at a heating rate of 10 K/min, is essentially as shown in Figure 4, and/or
> a TGA curve showing a mass loss of not more than 0.8% by weight, preferably of not more than 0.7% by weight, based on the weight of the cocrystal, when heated from 20 to 160°C at a rate of 10 K/min; and/or
> a TGA curve that, when measured at a heating rate of 10 K/min, is essentially as shown in Figure 5; and/or
> a combination of any two or more of the above.

The vorasidenib L-tartaric acid cocrystal of the present invention, particularly Form 3:
> shows excellent solubility in physiologically relevant media, e.g., a superior solubility in aqueous medium compared to the vorasidenib citric acid cocrystal Type A.
> is anhydrous and/or non-hygroscopic and/or non-solvated, thus being useful for pharmaceutical formulation.
> shows good flowability, high bulk density and/or good compressibility, attributes allowing for a robust formulation and ensuring a reliable safety and efficacy profile of a drug product during the whole shelf-life of the product.
> is thermally stable, as determined by DSC, i.e., it does not undergo phase transformations or decomposition until it melts at about 167 °C.

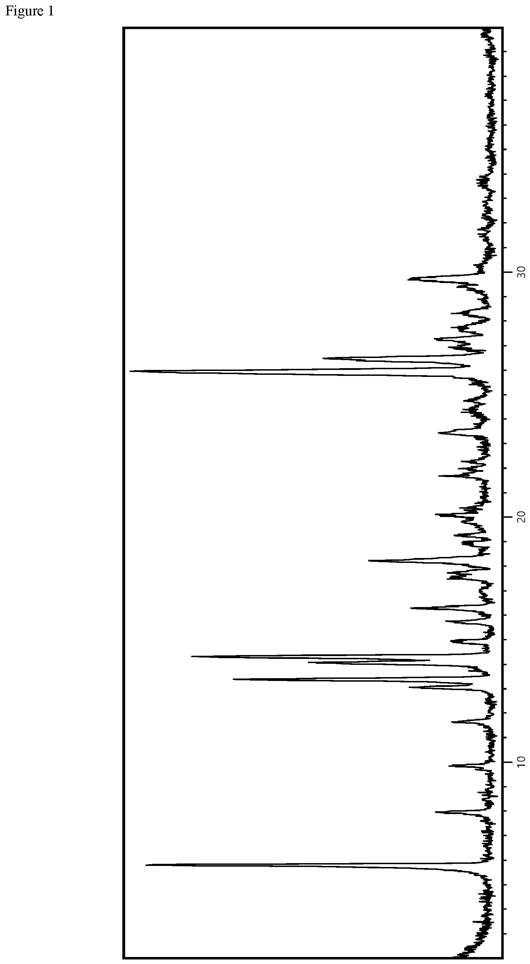

In another preferred embodiment thereof, the first aspect of the invention relates to a cocrystal of vorasidenib and L-tartaric acid, hereinafter Form 1, characterized by a PXRD essentially as shown in figure 1, when measured at RT with Cu-Kalpha_{1,2} radiation having a wavelength of 0.15419 nm.

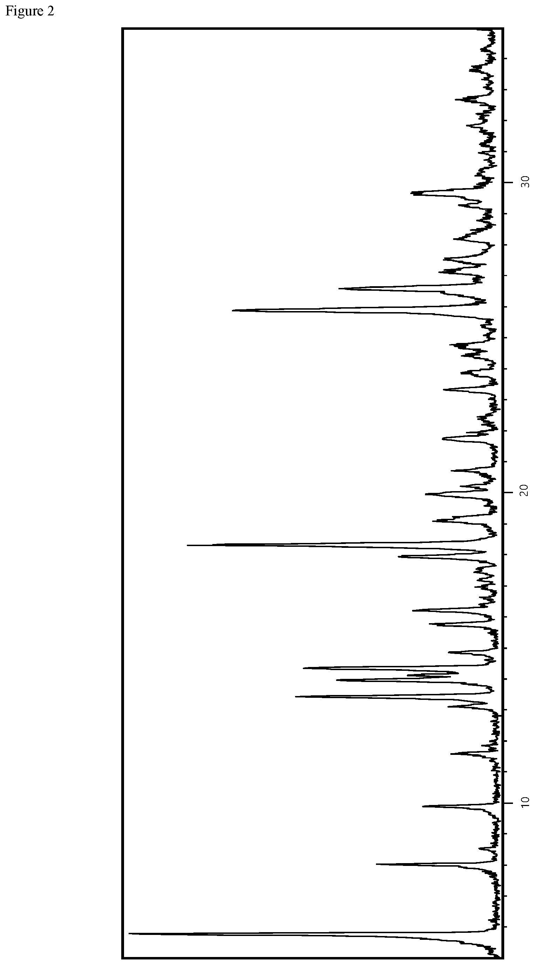

In another preferred embodiment thereof, the first aspect of the invention relates to a cocrystal of vorasidenib and L-tartaric acid, hereinafter Form 2, characterized by a PXRD essentially as shown in figure 2, when measured at RT with Cu-Kalpha_{1,2} radiation having a wavelength of 0.15419 nm.

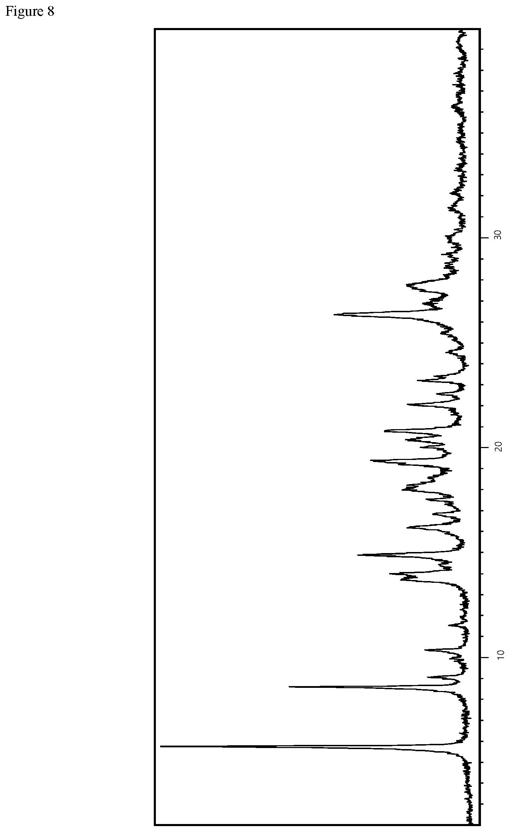

In another preferred embodiment thereof, the first aspect of the invention relates to a cocrystal of vorasidenib and L-tartaric acid, hereinafter Form 4, characterized by a PXRD essentially as shown in figure 8, when measured at RT with Cu-Kalpha_{1,2} radiation having a wavelength of 0.15419 nm.

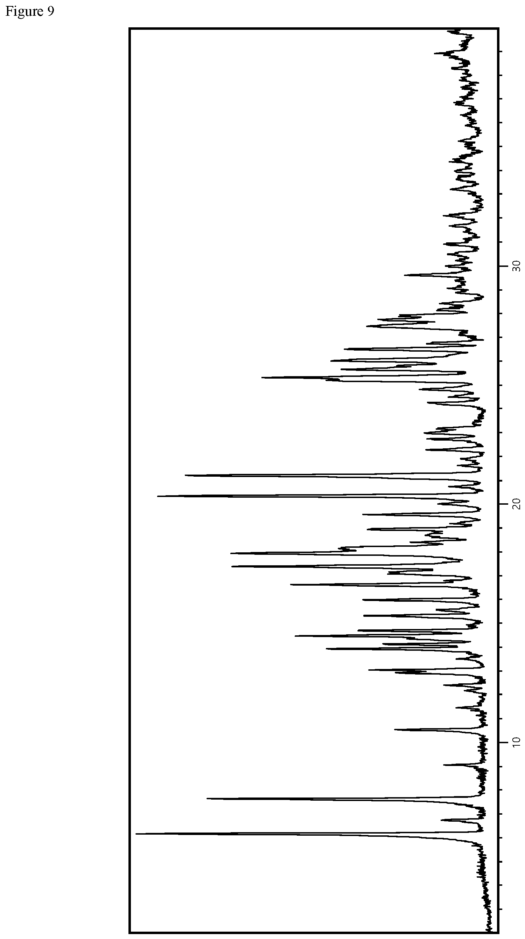

In another preferred embodiment thereof, the first aspect of the invention relates to a cocrystal of vorasidenib and L-tartaric acid, hereinafter Form 5, characterized by a PXRD essentially as shown in figure 9, when measured at RT with Cu-Kalpha_{1,2} radiation having a wavelength of 0.15419 nm.

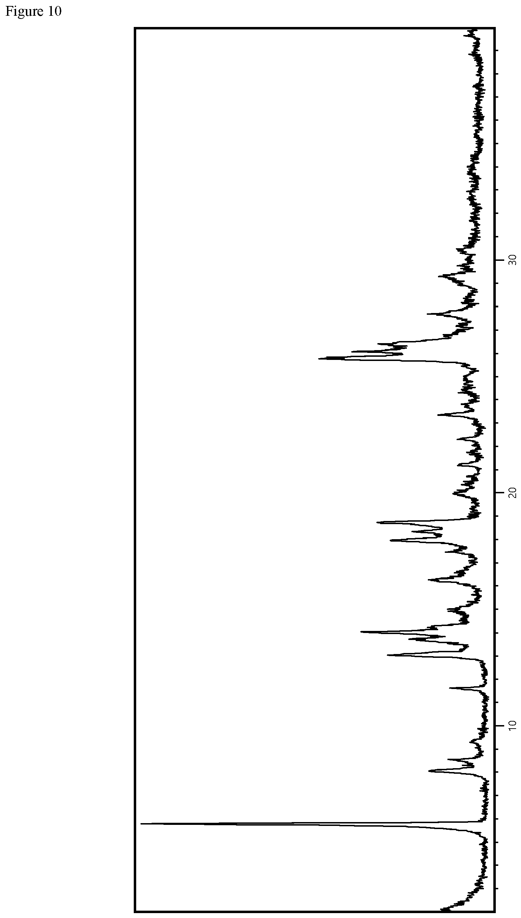

In another preferred embodiment thereof, the first aspect of the invention relates to a cocrystal of vorasidenib and L-tartaric acid, hereinafter Form 6, characterized by a PXRD essentially as shown in figure 10, when measured at RT with Cu-Kalpha_{1,2} radiation having a wavelength of 0.15419 nm.

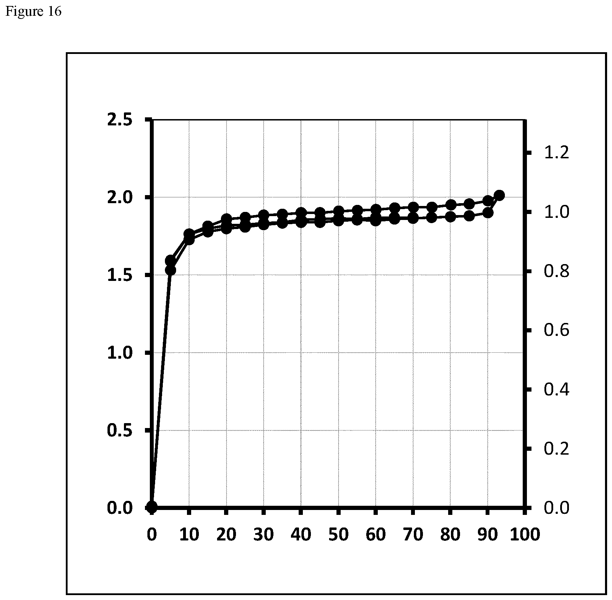

In another preferred embodiment thereof, the first aspect of the invention relates to a cocrystal of vorasidenib and tartaric acid (namely D/L-tartaric acid), preferably a cocrystal of vorasidenib and tartaric acid which is a hydrate and/or an ethanol solvate, more preferably a cocrystal of vorasidenib and tartaric acid which is a monohydrate and/or a mono-ethanol solvate. Even more preferably said cocrystal of vorasidenib and tartaric acid is characterized by a PXRD essentially as shown in figure 16, when measured at RT with Cu-Kalpha_{1,2} radiation having a wavelength of 0.15419 nm.

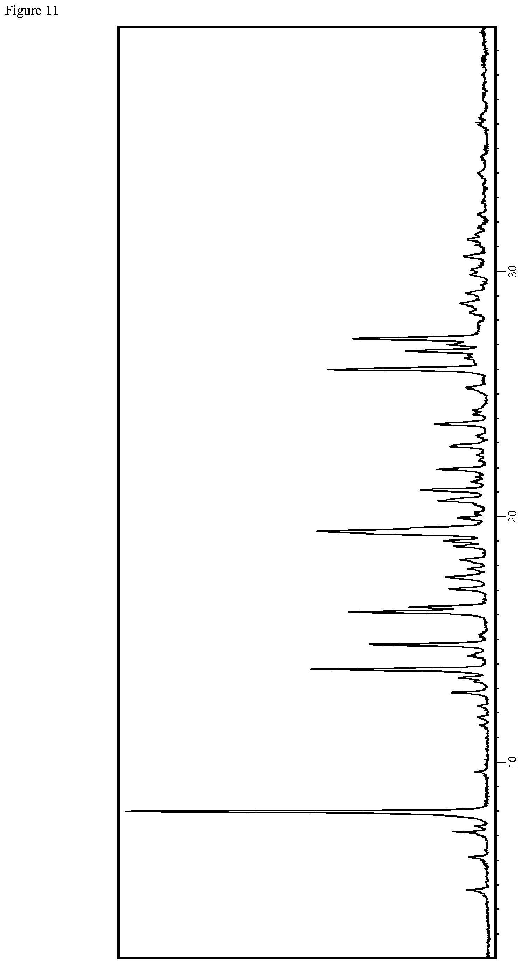

In another preferred embodiment thereof, the first aspect of the invention relates to a cocrystal of vorasidenib and malonic acid. More preferably said cocrystal of vorasidenib and tartaric acid is characterized by a PXRD essentially as shown in figure 11, when measured at RT with Cu-Kalpha_{1,2} radiation having a wavelength of 0.15419 nm.

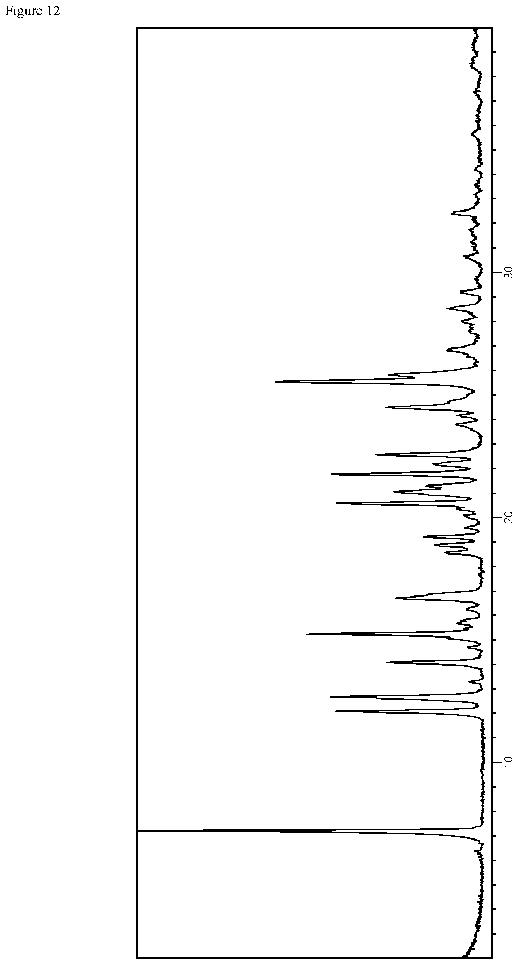

In another preferred embodiment thereof, the first aspect of the invention relates to a cocrystal of vorasidenib and glutaric acid. More preferably said cocrystal of vorasidenib and tartaric acid is characterized by a PXRD essentially as shown in figure 12, when measured at RT with Cu-Kalpha_{1,2} radiation having a wavelength of 0.15419 nm.

In a second embodiment thereof, the first aspect of the invention relates to cocrystal comprising vorasidenib, as the active pharmaceutical ingredient, and an unsaturated dicarboxylic acid having E-configuration at the double bond, preferably an (E)-unsaturated C2-C6 dicarboxylic acid, more preferably an (E)-unsaturated C2-C4 dicarboxylic acid, even more preferably fumaric acid, as the co-former.

In a preferred embodiment thereof, the first aspect of the invention relates to a cocrystal of vorasidenib and fumaric acid, preferably a cocrystal of vorasidenib and fumaric acid which is a hydrate.

In a particularly preferred embodiment thereof, said hydrate cocrystal of vorasidenib and fumaric is characterized by formula (III), 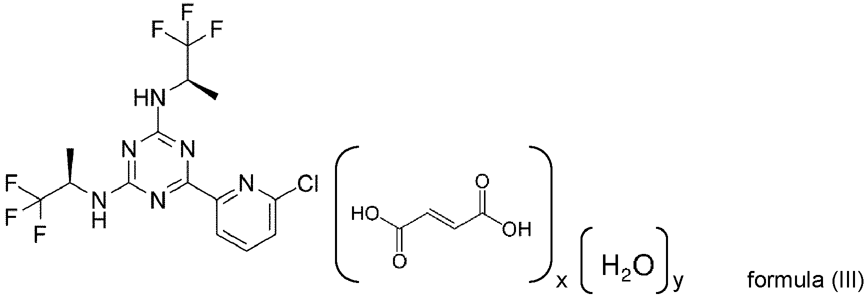 wherein x and y, independently of each other, are from 0.2 to 0.8, preferably from 0.3 to 0.7, more preferably from 0.4 to 0.6, most preferably 0.5.

Preferably the cocrystal of vorasidenib and fumaric acid, more preferably the cocrystal (III), is characterized by a PXRD comprising any one of the following reflections:
❖ (5.6 + 0.2) °, (9.5 + 0.2)° and (25.9 + 0.2)°; or
❖ (5.6 + 0.2) °, (9.5 + 0.2)°, (14.3 + 0.2)° and (25.9 + 0.2)°; or
❖ (5.6 + 0.2) °, (9.5 + 0.2)°, (13.7 + 0.2)°, (14.3 + 0.2)° and (25.9 + 0.2)°; or
❖ (5.6 + 0.2) °, (9.5 + 0.2)°, (13.7 + 0.2)°, (14.3 + 0.2)°, (21.6 + 0.2)° and (25.9 + 0.2)°; or
❖ (5.6 + 0.2) °, (9.5 + 0.2)°, (13.7 + 0.2)°, (14.3 + 0.2)°, (21.6 + 0.2)°, (25.9 + 0.2)° and (26.4 + 0.2)°; or
when measured at RT with Cu-Kalpha1 radiation having a wavelength of 0.15419 nm.

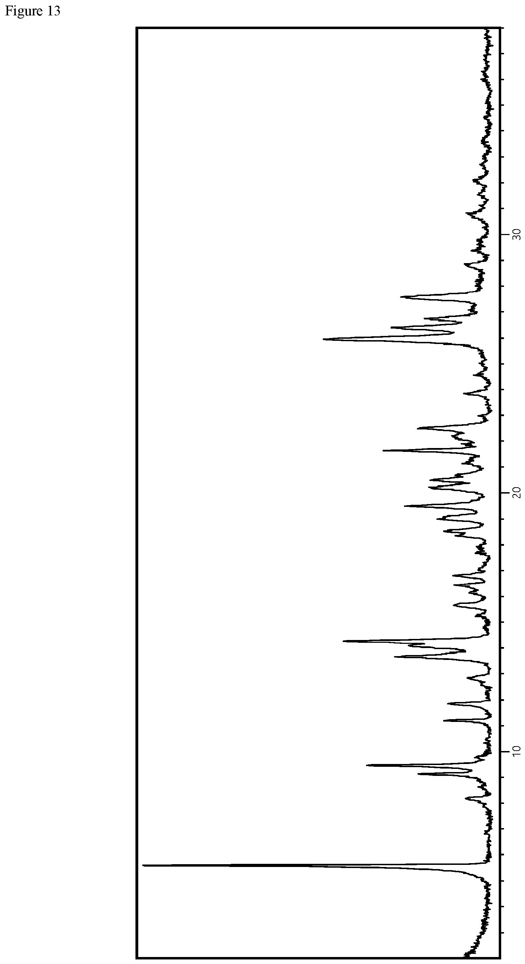

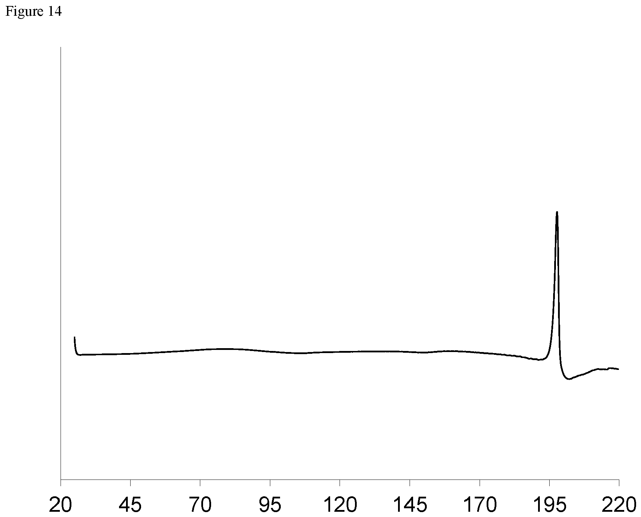

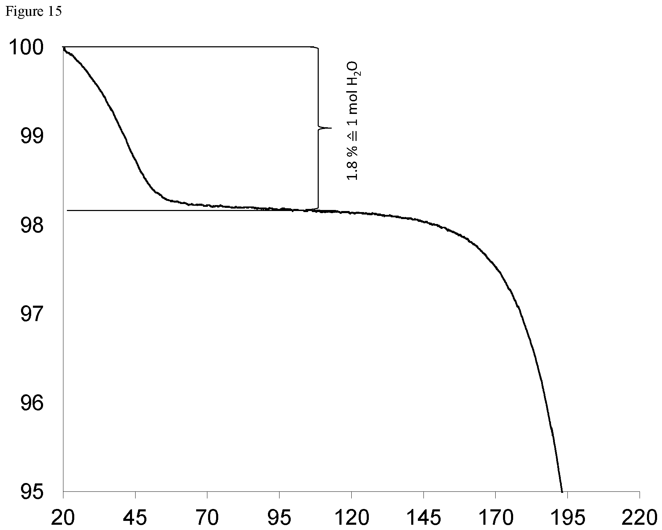

More preferably said vorasidenib fumaric acid cocrystal, preferably the cocrystal (III), is further characterized by at least one of the following features:
> a PXRD essentially as shown in Figure 13, when measured at RT with Cu-Kalpha_{1,2} radiation having a wavelength of 0.15419 nm, and/or
> a DSC curve comprising an endothermic peak, preferably a single endothermic peak, having an onset temperature of (196 ± 5) °C, preferably of (196 ± 3) °C, more preferably of (196 ± 2) °C, even more preferably of (196 ± 1) °C, when measured at a heating rate of 10 K/min in a perforated capsule, and/or
> a DSC trace that, when measured at a heating rate of 10 K/min, is essentially as shown in Figure 14, and/or
> a TGA curve showing a mass loss of not more than 1.8% by weight, when heated from 20 to 160°C at a rate of 10 K/min; and/or
> a TGA curve that, when measured at a heating rate of 10 K/min, is essentially as shown in Figure 15 and/or
> a DVS curve showing a water content of not more than 2.0% by weight, measured between 10 to 90 % RH, at a RH step of 5% RH; and/or
> a DVS curve that, when measured at a RH step of 5% RH, is essentially as shown in Figure 16 and/or
> a combination of any two or more of the above.

Fumaric acid cocrystal of vorasidenib, preferably cocrystal (III), is characterized by a set of favorable physicochemical properties, including high crystallinity, low hygroscopicity, and favorable bioavailability.

In a second embodiment thereof, the first aspect of the invention relates to a crystalline hydrate form of vorasidenib comprising less than 2.8 molecules of water per molecule of vorasidenib, preferably comprising from 0.5 to 2.5 moles of water per mole of vorasidenib, more preferably comprising from 1.0 to 2.2 moles of water per mole of vorasidenib, even more preferably comprising from 1.5 to 2.1 moles of water per mole of vorasidenib, most preferably to a dihydrate form of vorasidenib.

In a preferred embodiment thereof, the first aspect of the invention relates to a cocrystal (as defined in any of the embodiments described above) and / or crystalline hydrate of vorasidenib (as defined above), which is essentially free of any other cocrystals and/or solid-state forms of vorasidenib.

In a third aspect thereof, the present invention relates to a process for the preparation of a cocrystal of vorasidenib and a dicarboxylic acid, as defined hereinabove, said process comprising:
(i) dispersing vorasidenib and a dicarboxylic acid in at least one organic solvent;
(ii) mix the dispersion obtained in (i) for at least 15 hours so as to cause separation from the mother liquor of at least a portion of vorasidenib in the form of a cocrystal with the dicarboxylic acid;
(iii) optionally, separating the cocrystal obtained in step (ii);
(iv) optionally, washing the cocrystal isolated in step (iii);
(v) optionally, drying the cocrystal obtained in step (iii) or (iv);
(vi) optionally, dissolve the cocrystal obtained in step (iii), (iv) or (v) in at least one protic polar solvent; and
(vii) optionally, precipitate at least a portion of vorasidenib in the form of a cocrystal with L-tartaric acid.

According to a first embodiment of this aspect of the invention, a dicarboxylic acid is used in step (i), preferably a saturated or (E)-unsaturated C2-C6 dicarboxylic acid, more preferably a saturated or (E)-unsaturated C2-C4 dicarboxylic acid, even more preferably tartaric acid (e.g., D-tartaric acid and/or L-tartaric acid), glutaric acid, malonic acid and/or fumaric acid.

Preferably the molar ratio of vorasidenib and dicarboxylic acid used in step (i) is from 2.0 : 0.8 to 1.0 : 1.2, more preferably from 2.0 : 0.9 to 1.0 : 1.1, even more preferably from 2.00 : 0.95 to 1.0 : 1.05, most preferably the molar ratio is from 2.0 : 1.0 to 1.0 : 1.0.

More preferably, when the dicarboxylic acid is tartaric acid (e.g., L-tartaric and/or D-tartaric acid) or fumaric acid, the molar ratio of vorasidenib and dicarboxylic acid is from 2.0 : 0.8 to 2.0 : 1.2, more preferably from 2.0 : 0.9 to 2.0 : 1.1, even more preferably from 2.00 : 0.95 to 2.00 : 1.05, most preferably the molar ratio is 2.0 : 1.0.

Alternatively, when the dicarboxylic acid is malonic acid or glutaric acid, the molar ratio of vorasidenib and dicarboxylic acid is from 1.0 : 0.8 to 1.0 : 1.2, more preferably from 1.0 : 0.9 to 1.0 : 1.1, even more preferably from 1.00 : 0.95 to 1.00 : 1.05, most preferably the molar ratio is 1.0 : 1.0.

Organic solvents suitable for step (i) include nonpolar solvents, polar aprotic/protic solvents, and combinations thereof. Non-limiting examples of suitable nonpolar solvents include toluene, tert-butyl methyl ether, di-tert-butyl ether, diethyl ether, diisopropyl ether, cyclopropyl methyl ether, and combinations thereof. Suitable aprotic solvents include, for example, acetonitrile, methyl acetate, ethyl acetate, isopropyl acetate, n-butyl acetate, isobutyl acetate, tetrahydrofuran, 2-methyl tetrahydrofuran, and combinations thereof. In a preferred embodiment, the least one solvent is diisopropyl ether, acetonitrile, or a mixture thereof. Even more preferably the least one solvent is a mixture comprising diisopropyl ether and acetonitrile. Non-limiting examples of suitable polar protic solvents include water, methanol, ethanol, 1-butanol, and combinations thereof. In a preferred embodiment the polar protic solvent used in step (i) comprises methanol or ethanol.

The volume of the at least solvent used in step (i) can vary in a very wide range; preferably, it is from 0.01 mL to 10 mL, more preferably from 0.5 to 4 mL, per gram of vorasidenib.

Vorasidenib suitable to be used in step (i) is commercially available; alternatively, it can be prepared according to standard techniques in organic synthesis, e.g., according to the procedures described in US 2015/0018328 A1 or WO 2019/090059 A1.

Step (ii) include mixing the dispersion obtained in step (i) for at least 15 hours so as to cause precipitation of at least a portion of vorasidenib in cocrystal form or in hydrate form. Preferably, the dispersion prepared in step (i) is maintained under stirring in step (ii) from about 15 hours to 30 days, preferably from about 20 hours to 20 days, more preferably from about 24 hours to 10 days.

Preferably step (ii) involves stirring the dispersion prepared in step (i) at a temperature ranging between 10 and 50 °C, more preferably between 10 and 30 °C.

Once obtained, the vorasidenib cocrystal of step (ii) may be optionally separated from the mother liquor, in step (iii), using known techniques, such as filtration, centrifugation, solvent evaporation or decantation, more preferably by filtration or centrifugation.

Optionally, in step (iv), the cocrystal isolated in (iii) is washed with at least one solvent, preferably the same solvent or solvents mixture used in step (i).

In a preferred embodiment of this aspect of the invention, the cocrystals obtained in step (iii) or isolated in (iv) may be dried. Drying may be performed:
> at a temperature from about 20 to about 80 °C, preferably from about 20 to about 40 °C, most preferably drying is performed at RT;
> for a period from about 1 to about 72 hours, preferably from about 2 to about 48 hours, more preferably of from about 4 to about 24 hours, most preferably of from about 6 to about 18 hours; and/or
> at ambient pressure and/or under reduced pressure.

Preferably, drying step (v) is performed at a pressure of about 100 mbar or less, more preferably of about 50 mbar or less, most preferably of about 30 mbar or less, for example a pressure of about 25 mbar. In another embodiment, drying may be performed by storing the solid material over P₂O₅.

The cocrystals obtained in step (iii), isolated in (iv) or dried in (v) may be dissolved, in step (vi), in at least one protic polar solvent.

Non-limiting examples of suitable polar protic solvents include water, methanol, ethanol, 1-butanol, and combinations thereof. In a preferred embodiment the polar protic solvent used in step (vi) comprises 1-butanol.

The volume of the at least solvent used in step (vi) can vary in a very wide range; preferably, it is from 0.01 mL to 10 mL, more preferably from 0.5 to 4 mL, per gram of vorasidenib.

In step (vii), "precipitating" comprises evaporating a portion of the solvent from the solution obtained in step (vi) so as to cause precipitation of vorasidenib L-tartaric acid cocrystal, preferably vorasidenib L-tartaric acid cocrystal Form 6. Without intending to be bound by any theory, evaporating solvent from the solution may cause precipitation of the co-crystal and/or solid-state form of the invention by increasing its concentration in the solution (to its saturation point).

Evaporation may be performed at a temperature from about 20 to about 80 °C, preferably from about 20 to about 40 °C, most preferably evaporation is performed at RT. Evaporation may be performed at ambient pressure and/or under reduced pressure. Preferably, evaporation is performed under ambient conditions.

Once obtained, the vorasidenib L-tartaric acid cocrystal of step (vii) may optionally be separated from the mother liquor, washed and dried according to procedures equivalent to those described above in respect of step (iii), (iv) or (v).

In a further aspect thereof, the present invention relates to a process for the preparation of a crystalline hydrate form of vorasidenib comprising less than 2.8 molecules of water per molecules of vorasidenib, said process comprising:
(viii) disperse the cocrystal obtained in step (iii), (iv), (v) or (vii) in water or a solvent mixture comprising water;
(ix) maintain the dispersion obtained in (viii) under stirring for at least 5 minutes so as to cause separation from the mother liquor of at least a portion of vorasidenib in a hydrate form.

Preferably, step (viii) involves dispersing the cocrystal of step (iii), (iv), (v) or (vii), preferably the cocrystal obtained in step (iii), (iv) or (v), in water or a solvent mixture comprising water at a temperature from 10 to 50 °C, more preferably from 10 to 30 °C.

The volume of water or the solvent mixture comprising water used in step (viii) can range in very wide range; preferably, it is from 0.01 mL to 20 mL, more preferably from 5 mL to 15 mL, per gram of vorasidenib cocrystal.

The dispersion prepared in step (viii) may be stirred for a period from about 10 minutes to about 60 minutes, preferably from about 15 minutes to about 30 minutes, more preferably from about 20 minutes to about 25 minutes.

Once obtained, the hydrate form of vorasidenib may be separated from the mother liquor, washed and dried according to procedures equivalent to those described above in respect of step (iii), (iv) or (v).

Seed crystals may be added to promote crystallization during step (ii), (vii) or (ix). Seeding may be employed to control growth of the cocrystal and /or solid-state form of the invention or to control their particle size distribution.

### Pharmaceutical compositions and medical use

In a further aspect thereof, the present invention relates to the use of the cocrystal and/or solid-state form, as defined in any one of the aspects and embodiments described above, for the preparation of a pharmaceutical composition.

In a further aspect, the present invention relates to a pharmaceutical composition comprising the cocrystal and/or solid-state form, as defined in any one of the aspects and embodiments described above, preferably in an effective amount, and at least one pharmaceutically acceptable excipient.

Preferably, the pharmaceutical composition of the present invention is an oral solid dosage form, more preferably a tablet, even more preferably a film-coated tablet, i.e., a tablet comprising at least one core and a coating.

In another aspect thereof, the present invention relates to the use of a vorasidenib cocrystal and/or solid-state form, as defined hereinabove, and/or the compositions comprising it as a medicament.

In yet another aspect, the present invention relates to the cocrystal and/or solid-state form of vorasidenib, as defined in any one of the aspects and embodiments described above, for use in the treatment of an IDH1/IDH2-associated disease, preferably any disease, disorder or condition that can be prevented, ameliorated, or cured by modulating IDH activity.

In a further aspect thereof, the present invention relates to use of the cocrystal and/or solid-state form (as defined in any of the embodiments described hereinabove), for the manufacture of a medicament for the treatment of an IDH1/IDH2-associated disease, preferably a disease, disorder or condition that can be prevented, ameliorated, or cured by modulating IDH activity. A preferred embodiment of this aspect of the invention relates to the use of the cocrystal and/or solid-state form (as defined hereinabove), for the manufacture of a medicament for the treatment of cancer, preferably of malignant glioma and/or myoblastoma.

### EXAMPLES

The following non-limiting examples are illustrative for the disclosure and are not to be construed as to be in any way limiting for the scope of the invention.

The instruments and methods used to characterize the obtained solid form are as follows:
Powder X-ray diffraction patterns (PXRD) were performed with a X'Pert PRO diffractometer (PANalytical, Almelo, The Netherlands) equipped with a θ/θ coupled goniometer in transmission geometry, programmable XYZ stage with well plate holder, Cu-Kα1,2 radiation source (wavelength 0.15419 nm) with a focussing mirror, a 0.5° divergence slit, a 0.02° soller slit collimator and a 0.5° anti-scattering slit on the incident beam side, a 2 mm anti-scattering slit, a 0.02° Soller slit collimator, a Ni-filter and a solid-state PIXcel1D detector on the diffracted beam side. The patterns were recorded at a tube voltage of 40 kV, tube current of 40 mA, applying a stepsize of 0.013° 2Θ with 40s per step in the angular range of 2° to 40° 2θ. A typical precision of the 2-Theta values is in the range of ± 0.2° 2-Theta, preferably of ± 0.1° 2-Theta, more preferably of ± 0.05° 2-Theta.

Single crystal diffraction data were collected with a Xcalibur Gemini ultra diffractometer (Rigaku Oxford Diffraction) equipped with a Ruby CCD detector and a 4-circle kappa-goniometer using Cu-Kα or Mo-Kα radiation equipped with an Oxford Cryosystems 700 series Cryostream Plus cooler. The cell refinement and the data reduction were performed with CrysAlis PRO (Rigaku Oxford Diffraction, 2020).

Differential scanning calorimetry (DSC) was performed with a DSC 7 (Perkin-Elmer, Norwalk, Ct., USA) using the Pyris 8.0 software. Approximately 1 to 5 ± 0.0005 mg sample (using a UM3 ultramicrobalance, Mettler, Greifensee, CH) were weighed into Al-Pans (30 µL) and sealed with a cover (closed capsule), which was optionally perforated by a needle (perforated capsule). Dry nitrogen was used as the purge gas (purge: 20 mL min⁻¹), a heating rate of 10 K min⁻¹ was applied. The instrument was calibrated for temperature with pure benzophenone (mp. 48.0 °C) and caffeine (236.2 °C), and the energy calibration was performed with indium (mp. 156.6 °C, heat of fusion 28.45 J g⁻¹).

Thermogravimetric analysis was carried out with a TGA-7 system (Perkin-Elmer, Norwalk, Ct., USA) using the Pyris 8.0 software. A sample amount of 1 to 10 mg was weighed into a Platinum-sample holder (50 µL). A heating rate of 10 K min⁻¹ was applied and dry nitrogen was used as the purge gas (sample purge: 20 mL min⁻¹, balance purge: 40 mL min⁻¹). Temperature calibration was performed with ferromagnetic materials (Alumel and Ni, Curie-point standards, Perkin-Elmer).

The moisture sorption isotherms were recorded with a SPS-11 moisture sorption analyzer (MD Messtechnik, Ulm, Germany). The measurement cycle was started at 70 or 60 % relative humidity (RH), decreased in 5 % steps down to 0 % RH, increased up to 95% RH in 5% steps, decreased in 5% steps down to 0 % RH. The equilibrium condition for each step was set to a mass constancy of ±0.002 % over 40 min.

The UV spectra were obtained with a Shimadzu UV-1800 double beam photometer with a wavelength range from 190 nm to 1100 nm and a spectral bandwidth of 1 nm. The solubility concentration was calculated based on the absorbance at 282 nm. All measurements have been performed in a 1 cm quartz glass cuvette. For the generation of the standard curves, stock solutions with a defined sample weight of solid forms in phosphate buffer (pH 6.8, 0.01 mol/L + 0.003% Tween80) and 10% methanol were prepared. Different concentrations were prepared by diluting the stock solutions. The absorbance of the stock solutions and the dilutions was measured at 282 nm wavelength and the standard curves were generated by plotting the concentration against the absorbance.

### Example 1: Preparation of the vorasidenib L-tartaric acid cocrystal Form 1

400.0 mg of vorasidenib Free Form A (prepared according to the procedure described in US 2015/0018328 A1 or WO 2019/090059 A1) and 105 mg L-tartaric acid were suspended in 1.0 mL ethanol and stirred for 30 days at 15-25 °C. The solid was collected by filtration through a Büchner funnel and dried at room temperature for 6 hours. The obtained product was analyzed by PXRD, obtaining the diffractogram substantially as depicted in Figure 1. The single crystal structure elucidation revealed a co-crystal of vorasidenib:L-tartaric acid:water:ethanol in a stoichiometric ratio of 2:1:2:1.

### Example 2: Preparation of the vorasidenib L-tartaric acid cocrystal Form 2

A suspension of 200 mg of vorasidenib Free Form A (prepared according to the procedure described in US 2015/0018328 A1 or WO 2019/090059 A1.) and 72.4 mg of L-tartaric acid was stirred in 0.3 mL of ethanol at 10-25 °C for 26 hours. The obtained solid was filtered and washed with 0.2 mL of water to remove the excess of L-tartaric acid. The obtained product was analyzed by PXRD, obtaining the diffractogram substantially as depicted in Figure 2. Single crystal structure elucidation revealed a co-crystal of vorasidenib: L-tartaric acid:water in a stoichiometric ratio of 2:1:3.4.

### Example 3: Preparation of the vorasidenib L-tartaric acid cocrystal Form 3

775.0 mg of vorasidenib Free Form A (prepared according to the procedure described in US 2015/0018328 A1 or WO 2019/090059 A1), and 140.2 mg of L-tartaric acid was suspended in 1 mL of acetronitrile/diisopropyl ether (1:3 v/v). The resulting suspension was stirred at 10-30 °C for three days. The solid was collected by filtration through a Büchner funnel and dried at room temperature for 6 hours. The obtained product was analyzed by PXRD, DSC and TGA.

A representative diffractogram of the Form 3 is depicted in Figure 3, while the corresponding peaks list from 2 to 30° 2-Theta is provided in Table 1.

**Table 1: Reflection (peak) positions of the Form 3 in the range of from 2 to 30° 2-Theta.**

| **Position [2-Theta °]** | **Relative Intensity** [**%**] |
|---|---|
| 5.8 | 61.7 |
| 8.6 | 26.5 |
| 10.2 | 44.2 |
| 10.5 | 9.6 |
| 11.6 | 4.2 |
| 13.6 | 85.7 |
| 13.7 | 100.0 |
| 14.2 | 4.4 |
| 14.7 | 21.9 |
| 15.8 | 29.7 |
| 16.0 | 23.0 |
| 17.3 | 1.5 |
| 18.0 | 2.7 |
| 18.3 | 6.3 |
| 18.4 | 18.0 |
| 19.1 | 42.1 |
| 19.6 | 6.2 |
| 19.9 | 23.1 |
| 20.3 | 19.5 |
| 20.5 | 36.1 |
| 21.2 | 14.2 |
| 22.9 | 1.4 |
| 23.2 | 11.4 |
| 23.6 | 7.4 |
| 24.5 | 2.4 |
| 25.1 | 8.4 |
| 25.4 | 28.0 |
| 26.0 | 15.0 |
| 26.4 | 2.5 |
| 26.9 | 27.6 |
| 26.9 | 23.6 |
| 27.4 | 32.3 |
| 27.6 | 16.3 |
| 28.0 | 1.8 |
| 28.2 | 2.9 |
| 29.2 | 17.4 |
| 29.4 | 7.9 |

The product was also subjected to DSC and TGA analyses, which gave as results the traces shown in Figures 4 and 5, respectively.

### Example 4: Preparation of the seed of Form 3

A grinding jar was charged with 25.0 mg of vorasidenib Free Form A (prepared according to the procedure described in US 2015/0018328 A1 or WO 2019/090059 A1) and 4.5 mg of L-tartaric acid. 0.1 mL of acetonitrile/diisopropyl ether (1:3 v/v) were added to the jar. The resulting mixture was ground in a Retsch mill at 15 Hz for 60 minutes. The obtained product was analyzed by XRPD, obtaining the diffractogram substantially as depicted in Figure 3.

### Example 5: Single crystal X-ray diffraction analysis of Form 3

Single crystals of the L-tartaric acid cocrystal Form 3 were obtained by slow evaporation of a solution of vorasidenib L-tartaric acid cocrystal Form 3 in acetonitrile/diisopropyl ether (1:3, v/v). The experiment details were as follows: 15.0 mg of vorasidenib L-tartaric acid cocrystal Form 3 were weighed into a 3 mL vial. 1 mL of solvent (acetonitrile/diisopropyl ether (1:3, v/v)) was added to the vial and the solids were dissolved by vortexing for 5 minutes. A pierced lid was placed on the 3 mL vial to let the solvent evaporate slowly. After two days, prismatic crystals of the L-tartaric acid cocrystal Form 3 were obtained.

The crystal data and structure refinement are listed in Table 2. In the crystal structure, the molar ratio of vorasidenib to L-tartaric acid is 2:1.

**Table 2: Crystal data and structure refinement for Form 3 single crystal**

| **Empirical formula** | | **(C₁₄ H₁₃ Cl F₆ N₆)₂·C₄ H₆ O₆** |
|---|---|---|
| | Crystal system | monoclinic |
| | Space group | C2 |
| | a [Å] | 20.509(9) |
| | b [Å] | 6.9858(11) |
| | c [Å] | 18.099(8) |
| | alpha [deg] | 90 |
| | beta [deg] | 122.43(6) |
| | gamma [deg] | 90 |
| | V [Å₃/cell] | 2188.8 (19) |
| | Dc [g cm⁻³] | 1.486 |
| | Z, Z' | Z:4, Z':1 |
| | R-Factor [%] | 6.81 |
| | Packing coefficient | 0.626889 |

### Example 6: Time-dependent solubility determination of Form 3

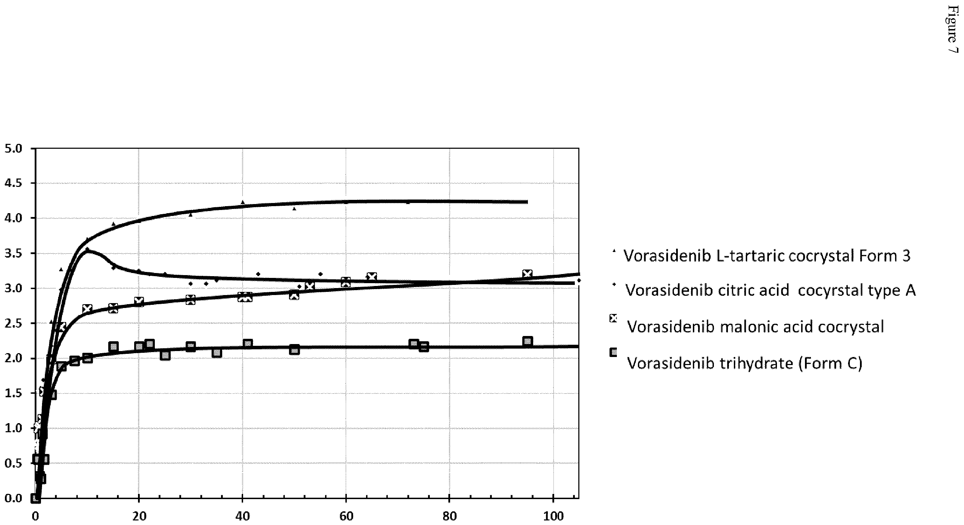

The phosphate buffer solution (pH 6.8, 0.01 mol/L + 0.003% Tween 80) containing 10% of methanol (v/v) was equilibrated in a water bath at a temperature of 25 °C. To obtain supersaturated solutions, the following amounts of vorasidenib solid forms were added to 65 mL of the equilibrated buffer solutions as follows: 6.2 mg of vorasidenib Free Form Type C (Trihydrate), 6.9 mg of vorasidenib malonic acid cocrystal, 6.6 mg of vorasidenib L-tartaric cocrystal Form 3, and 6.9 mg of vorasidenib citric acid cocrystal Type A. Vorasidenib malonic acid cocrystal transforms to a vorasidenib dihydrate within ten minutes. Samples were withdrawn at different time points and filtered through 0.45 µm membrane filters. The absorbance was measured with UV-Vis spectroscopy at 282 nm. The measurements were performed in triplicates. The calculated concentrations of the solid forms are shown in Figure 7.

The solubility data is listed in Table 3. The different solid-state forms of vorasidenib reach a saturation concentration after 30 to 40 minutes. Vorasidenib citric acid cocrystal Type A transforms to the vorasidenib dihydrate within one minute. Vorasidenib L-tartaric acid cocrystal Form 3 shows higher solubility than vorasidenib citric acid cocrystal Type A due to its higher stability and slower hydrate transformation.

**Table 3: Solubility data of vorasidenib solid-state forms.**

| **Solid-state Form** | **Mr [g/mol]** | **Molar solubility [mmol/l] (mean ± SD)** | **Mass solubility [mg/l] (mean ± SD)** |
|---|---|---|---|
| Vorasidenib malonic acid cocrystal | 450.78 | 0.0070 ± 0.0003 | 2.61 ± 0.04 |
| Vorasidenib Free Form Type C (Thhydrate) | 468.80 | 0.0046 ± 0.0002 | 1.67 ± 0.08 |
| Vorasidenib L-tartaric acid cocrystal Form 3 | 493.03 | 0.0084 ± 0.0002 | 3.49 ± 0.09 |
| Vorasidenib citric acid cocrystal Type A | 519.81 | 0.0060 ± 0.0002 | 2.51 ± 0.09 |

### Example 7: Preparation of the vorasidenib L-tartaric acid cocrystal Form 4

50 mg of vorasidenib L-tartaric acid cocrystal Form 2, prepared according to Example 2, was dried at 0% RH by storing the solid over P₂O₅ for 26 days. The dried solid was analyzed by PXRD, obtaining the diffractogram substantially as depicted in Figure 8.

### Example 8: Preparation of the vorasidenib L-tartaric acid cocrystal Form 5

194.9 mg of vorasidenib Free Form A (prepared according to the procedure described in US 2015/0018328 A1 or WO 2019/090059 A1) and 72.4 mg L-tartaric acid were stirred for six months in a water bath at 10-30 °C with once weekly addition of 0.2 mL of diisopropyl ether. The obtained material was stored at ambient conditions for three months. 0.1 mL of acetonitrile:diisopropyl ether (1:3 v/v) were added to the resulting solid and the obtained suspension was stirred at 10-30 °C for 10 hours. The obtained solid was analyzed by PXRD, obtaining the diffractogram substantially as depicted in Figure 9.

### Example 9: Preparation of the vorasidenib L-tartaric acid cocrystal Form 6

20.0 mg of vorasidenib Free Form A (prepared according to the procedure described in US 2015/0018328 A1 or WO 2019/090059 A1) and 7.2 mg of L-tartaric acid was suspended in 1 mL of 1-BuOH by stirring and heating at 50 °C for 15 minutes. To remove excess of undissolved L-tartaric acid, the mixture was filtered into an 8 mL vial with a perforated lid, and the obtained solution was placed at ambient conditions for evaporation for 30 days. The obtained solid was analyzed by PXRD, obtaining the diffractogram substantially as depicted in Figure 10.

### Example 10: Preparation of the vorasidenib malonic acid cocrystal

800.0 mg of vorasidenib Free Form A (prepared according to the procedure described in US 2015/0018328 A1 or WO 2019/090059 A1) and 200.7 mg of malonic acid were suspended in 4.2 mL of toluene and stirred at room temperature. After three days the obtained solid was filtered under reduced pressure with a Büchner funnel and dried at ambient conditions for one day. The obtained product was analyzed by PXRD, obtaining the diffractogram substantially as depicted in Figure 11. The single crystal structure was determined as a co-crystal of vorasidenib: malonic acid in a stoichiometric ratio of 1:1.

### Example 11: Preparation of the vorasidenib glutaric acid cocrystal

A saturated solution of glutaric acid in acetonitrile:diisopropyl ether was prepared by stirring 128 mg of glutaric acid in 2 mL of acetonitrile:diisopropyl ether (1:3 v/v) for one hour and subsequently filtered. 100.0 mg of vorasidenib Free Form A (prepared according to the procedure described in US 2015/0018328 A1 or WO 2019/090059 A1) and 31.9 mg of glutaric acid were suspended in 0.1 mL of the saturated glutaric acid solution and kept under stirring for 3 days at room temperature. The obtained product was analyzed by PXRD, obtaining the diffractogram substantially as depicted in Figure 12. The single crystal structure was determined as a co-crystal of vorasidenib:glutaric acid in a stoichiometric ratio of 1:1

### Example 12: Preparation of the vorasidenib fumaric acid cocrystal

600.0 mg of vorasidenib form A (prepared according to the procedure described in US 2015/0018328 A1 or WO 2019/090059 A1) and 84.0 mg of fumaric acid were suspended in 2.3 mL of methanol and stirred for 12 hours at room temperature. The obtained solid was filtered under reduced pressure with a Büchner funnel and dried at room temperature for 6 hours. The obtained product was analyzed by PXRD, obtaining the diffractogram substantially as depicted in Figure 13. The single crystal structure was determined as a co-crystal of vorasidenib:fumaric acid:water in a stoichiometric ratio of 2:1:1.

### Example 13: Preparation of the vorasidenib hydrate form

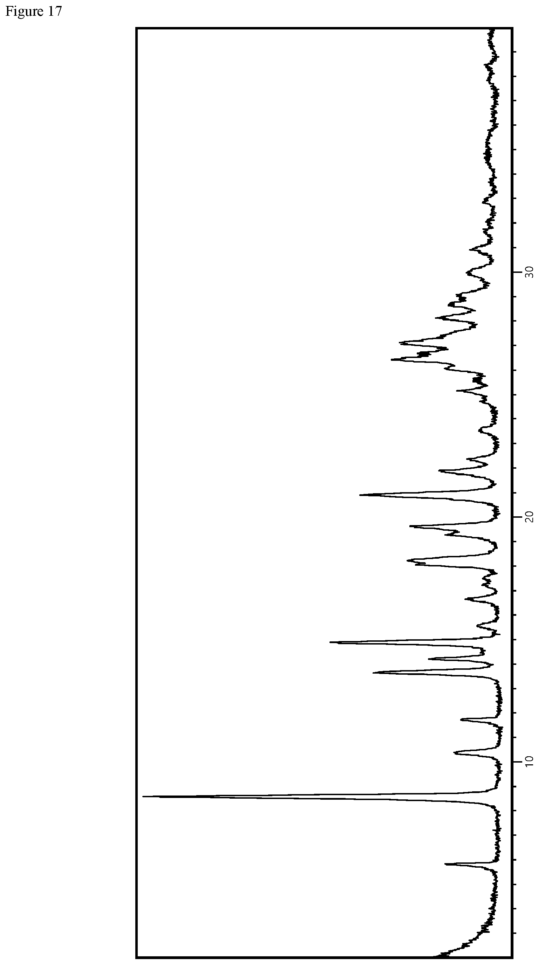

130 mg of vorasidenib malonic acid cocrystal, prepared according to Example 10, was suspended in 2 mL of water, and stirred at room temperature for 20 minutes. The obtained solid was analyzed by PXRD, obtaining the diffractogram substantially as depicted in Figure 17.

### Example 14: Preparation of the vorasidenib D/L-tartaric acid cocrystal

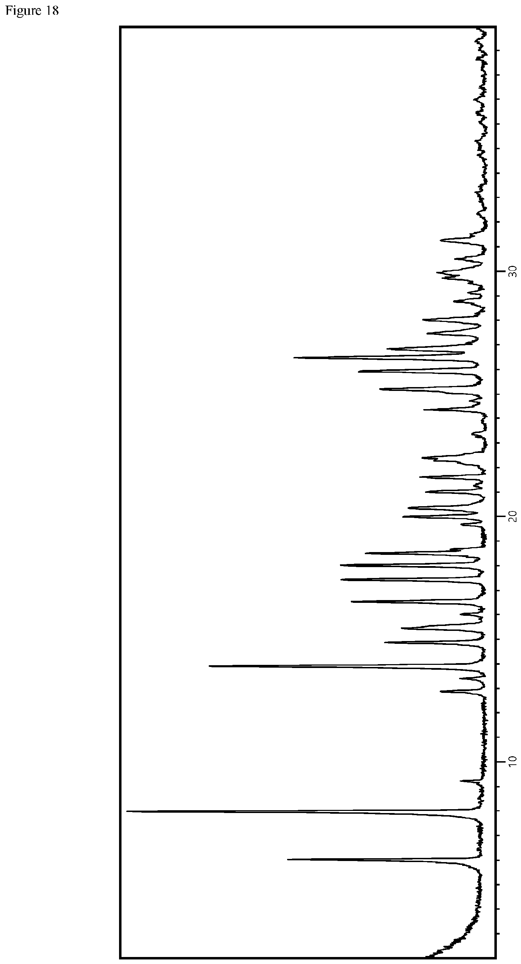

500.0 mg of vorasidenib form A (prepared according to the procedure described in US 2015/0018328 A1 or WO 2019/090059 A1) and 90.5 mg of D/L-tartaric acid were suspended in 0.8 mL ethanol and stirred for 2 days at 10-25 °C. A physical mixture of vorasidenib and D/L-tartaric acid was obtained, and 10 mg of D/L-tartaric acid was added to the mixture. After five days, a mixture of the co-crystal and co-former was obtained. The suspension was filtered under reduced pressure and the excess of co-former was removed by washing the solids with water. To the obtained mixture, 5 mg of D/L-tartaric acid and 0.5 mL ethanol were added. The slurry was stirred for another 9 days and filtered under reduced pressure using a Büchner filter and dried at ambient conditions. The obtained product was analyzed by PXRD, obtaining the diffractogram substantially as depicted in Figure 18. The single crystal structure was determined as a co-crystal of vorasidenib: D/L-tartaric acid:water:ethanol in a stoichiometric ratio of 2:1:1:1.

## Claims

1. A cocrystal comprising a compound of formula (I) 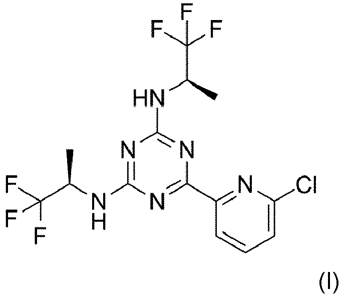 and a dicarboxylic acid.

2. The cocrystal according to claim 1, wherein the dicarboxylic acid is a C2-C6 saturated or a C2-C6 (E)-unsaturated dicarboxylic acid.

3. The cocrystal according to any one of claims 1 and 2, wherein the dicarboxylic acid is selected from the group consisting of tartaric acid, D-tartaric acid, L-tartaric acid., malonic acid, glutaric acid, and fumaric acid.

4. The cocrystal according to any one of claims 1 to 3, wherein the carboxylic acid is L-tartaric acid.

5. The cocrystal according to claim 4, **characterized in that** it is anhydrous and/or non-solvated and/or non-hygroscopic.

6. The cocrystal according to any one of claims 4 and 5, **characterized in that** it shows at least one of the following features (i) to (xii):
(i) an X-ray powder diffraction pattern, when collected with the Kα radiation of copper (λ = 1.5419 Å) and a temperature from 20 to 30°C, is essentially as shown in Figure 3; and/or
(ii) an X-ray powder diffraction pattern that, when collected with the Kα radiation of copper (λ = 1.5419 Å) and a temperature from 20 to 30 °C, shows main reflections 2-Theta at (5.8 + 0.2)°, (10.2 + 0.2)° and (13.7 + 0.2)°; and/or
(iii) an X-ray powder diffraction pattern that, when collected with the Kα radiation of copper (λ = 1.5419 Å) and a temperature from 20 to 30°C, shows main reflections 2-Theta at (5.8 + 0.2)°, (8.6 + 0.2)°, (10.2 + 0.2)°, (13.7 + 0.2)°; and/or
(iv) an X-ray powder diffraction pattern that, when collected with the Kα radiation of copper (λ = 1.5419 Å) and a temperature from 20 to 30 °C, shows main reflections 2-Theta at (5.8 + 0.2) °, (8.6 + 0.2)°, (10.2 + 0.2)°, (13.7 + 0.2)°, and (19.1 + 0.2)°; and/or
(v) an X-ray powder diffraction pattern that, when collected with the Kα radiation of copper (λ = 1.5419 Å) and a temperature from 20 to 30 °C, shows main reflections 2-Theta at (5.8 + 0.2)°, (8.6 + 0.2)°, (10.2 + 0.2) °, (13.7 + 0.2)°, (15.8 + 0.2)°and (19.1 + 0.2)°; and/or
(vi) an X-ray powder diffraction pattern that, when collected with the Kα radiation of copper (λ = 1.5419 Å) and a temperature from 20 to 30 °C, shows main reflections 2-Theta at (5.8 + 0.2)°, (8.6 + 0.2)°, (10.2 + 0.2)°, (13.7 + 0.2)°, (15.8 + 0.2)°, (19.1 + 0.2)° and (27.4 + 0.2)°; and/or
(vii)a monoclinic unit cell having space group C2 with the following parameters:
a = 20.509(9)
b = 6.9858(11)
c = 18.099(8)
beta = 122.43(6)°
when measured with single crystal X-ray diffraction at (173 ± 2) K with Cu-Kalpha_{1,2} radiation having a wavelength of 1.54184 Angstrom); and/or
(viii) a DSC trace comprising an endothermic peak having an onset at a temperature of (167 ± 5)°C, when measured at a heating rate of 10 K/min in a closed capsule; and/or
(ix) a DSC trace that, when measured at a heating rate of 10 K/min, is essentially as shown in Figure 4; and/or
(x) a TGA curve showing a mass loss of not more than 0.8% by based on the weight of the cocrystal, when heated from 20°C to 160°C at a rate of 10 K/min; and/or
(xi) a TGA curve that, when measured at a heating rate of 10 K/min, is essentially as shown in Figure 5; and/or
(xii)a combination of any two or more (i) - (xi).

7. The cocrystal according to any one of claims 1 to 6, wherein the molar ratio of vorasidenib to dicarboxylic acid is from 0.2 to 0.8.

8. The cocrystal according to any one of claims 1 to 3, wherein the carboxylic acid is fumaric acid.

9. The cocrystal according to claim 8, **characterized in that** it shows at least one of the following features (i) to (xi):
(i) an X-ray powder diffraction pattern, when collected with the Kα radiation of copper (λ = 1.5419 Å) and a temperature from 20 to 30°C, is essentially as shown in Figure 13; and/or
(ii) an X-ray powder diffraction pattern that, when collected with the Kα radiation of copper (λ = 1.5419 Å) and a temperature from 20 to 30 °C, shows main reflections 2-Theta at (5.6 + 0.2)°, (9.5 + 0.2)°, and (25.9 + 0.2)°; and/or
(iii) an X-ray powder diffraction pattern that, when collected with the Kα radiation of copper (λ = 1.5419 Å) and a temperature from 20 to 30°C, shows main reflections 2-Theta at (5.6 + 0.2)°, (9.5 + 0.2)°, (14.3 + 0.2)°, and (25.9 + 0.2)°; and/or
(iv) an X-ray powder diffraction pattern that, when collected with the Kα radiation of copper (λ = 1.5419 Å) and a temperature from 20 to 30 °C, shows main reflections 2-Theta at (5.6 + 0.2)°, (9.5 + 0.2)°, (13.7 + 0.2)°, (14.3 + 0.2)°, and (25.9 + 0.2)°; and/or
(v) an X-ray powder diffraction pattern that, when collected with the Kα radiation of copper (λ = 1.5419 Å) and a temperature from 20 to 30 °C, shows main reflections 2-Theta at (5.6 + 0.2)°, (9.5 + 0.2)°, (13.7 + 0.2)°, (14.3 + 0.2)°, (21.6 + 0.2)°, and (25.9 + 0.2)°; and/or
(vi) an X-ray powder diffraction pattern that, when collected with the Kα radiation of copper (λ = 1.5419 Å) and a temperature from 20 to 30 °C, shows main reflections 2-Theta at (5.6 + 0.2) °, (9.5 + 0.2)°, (13.7 + 0.2)°, (14.3 + 0.2)°, (21.6 + 0.2)°, (25.9 + 0.2)° and (26.4 + 0.2)°; and/or
(vii) a DSC trace comprising an endothermic peak having an onset at a temperature of (196 ± 5)°C, when measured at a heating rate of 10 K/min in a perforated capsule; and/or
(viii) a DSC trace that, when measured at a heating rate of 10 K/min, is essentially as shown in Figure 14; and/or
(ix) a TGA curve showing a mass loss of not more than 1.8% by based on the weight of the cocrystal, when heated from 20 to 160°C at a rate of 10 K/min; and/or
(x) a TGA curve that, when measured at a heating rate of 10 K/min, is essentially as shown in Figure 16; and/or
(xi) a combination of any two or more (i) - (x).

10. A process for preparing a cocrystal according to any one of claims 1 to 9 comprising:
(i) dispersing vorasidenib and a dicarboxylic acid in at least one organic solvent;
(ii) mix the dispersion obtained in (i) for at least 15 hours so as to cause separation from the mother liquor of at least a portion of vorasidenib in the form of a cocrystal with the dicarboxylic acid;
(iii) optionally, separating the cocrystal obtained in step (ii);
(iv) optionally, washing the cocrystal isolated in step (iii);
(v) optionally, drying the cocrystal obtained in step (iii) or (iv);
(vi) optionally, dissolve the cocrystal obtained in step (iii), (iv) or (v) in at least one protic polar solvent; and
(vii)optionally, precipitate at least a portion of vorasidenib in the form of a cocrystal with L-tartaric acid.

11. Use of a cocrystal according to any one of claims 1 to 9 for the preparation of a pharmaceutical composition.

12. Pharmaceutical composition comprising a cocrystal according to any one of claims 1 to 9 and at least one pharmaceutically acceptable excipient.

13. The cocrystal according to any one of claims 1 to 9 for use as a medicament.

14. The pharmaceutical composition according to claim 12 for use in therapy.

15. The cocrystal according to any one of claims 1 to 9 for use in treating cancer.
